# EUROPÄISCHE PATENTSCHRIFT

(11) **EP 0 690 030 B1**
(45) Veröffentlichungstag und Bekanntmachung des Hinweises auf die Patenterteilung: **07.05.1997**
(21) Anmeldenummer: 95250155.9
(22) Anmeldetag: 29.06.1995
(51) Int. Cl.: C03C 10/00, A61K 6/06

(54) **Leucithaltige Phosphosilikat-Glaskeramik**
Phosphosilicate glass ceramics containing leucite
Vitrocéramique de phosphosilicate avec du leucite

(30) Priorität: 01.07.1994 DE 4423793
(43) Veröffentlichungstag der Anmeldung: 03.01.1996
(73) Patentinhaber: IVOCLAR AG, FL-9494 Schaan (LI)
(72) Erfinder: Frank, Martin, FL-9494 Schaan (LI); Schweiger, Marcel, CH-7000 Chur (CH); Rheinberger, Volker, FL-9490 Vaduz (LI); Höland, Wolfram, FL-9494 Schaan (LI)
(74) Vertreter: UEXKÜLL & STOLBERG

(56) Entgegenhaltungen:
- EP-A- 0 272 745
- FR-A- 2 558 726
- GB-A- 2 199 027
- US-A- 3 907 577

## Beschreibung

Die Erfindung betrifft leucithaltige Phosphosilikat-Glaskeramiken und insbesondere leucithaltige Phosphosilikat-Glaskeramiken, die sich aufgrund ihrer mechanischen, optischen, chemischen, biologischen und ihrer Verarbeitungseigenschaften in ausgezeichneter Weise zur Verwendung in der Dentaltechnik eignen.

Phosphosilikat-Glaskeramiken stellen Werkstoffe dar, die hauptsächlich aus SiO₂ aufgebaut sind und die neben anderen Bestandteilen auch P₂O₅ enthalten. Sie weisen eine oder mehrere Kristallphasen sowie eine oder mehrere Glasphasen auf und werden aus einem kristallfreien P₂O₅-haltigen Silikat-Ausgangsglas durch gesteuerte Kristallisation erhalten.

Bei leucithaltigen Glaskeramiken führt die gesteuerte Kristallisation des Ausgangsglases, auch als Entglasung bezeichnet, zur Bildung von Leucitkristallen vom Typ K[AlSi₂O₆].

Aus dem Stand der Technik sind zum einen leucitfreie Phosphosilikat-Glaskeramiken und zum anderen leucithaltige Glaskeramiken und Keramiken bekannt, welche jedoch nicht von dem Phosphosilicat-System abgeleitet sind.

Leucitfreie Phosphosilikat-Glaskeramik für den Knochenersatz werden beispielsweise von Kokubo beschrieben (*Biomaterials* **12** (1991) 155). Sie weisen eine MgO-CaO-SiO₂-Glasmatrix mit darin homogen verteilten Apatit (Ca₁₀(PO₄)₆O, F₂)- und β-Wollastonit (CaO·SiO₂)-Kristallen auf. Die Glaskeramiken sind jedoch bioaktiv, d.h. sie besitzen eine besonders hohe Oberflächenreaktivität und bilden bei Reaktion mit Körperflüssigkeiten auf ihrer Oberfläche Kristalle aus. Diese Eigenschaft befähigt sie dazu, mit dem lebenden Knochen eine direkte, nahezu bindegewebsfreie Bindung einzugehen. In der Dentaltechnik, wie beispielsweise bei der Anwendung von Glaskeramiken als Dentalkrone oder -brücke, ist jedoch eine solche bioaktive Oberflächenreaktion unerwünscht.

Weitere leucitfreie bioaktive Glaskeramiken sind aus der DE-PS 33 06 648 und der DE-OS 39 39 831 bekannt. Diese Glaskeramiken weisen als Hauptkristallphasen Glimmer und Apatit auf. Eine Kombination von Eigenschaften, wie sie für Dentalmaterialien und Dentalprodukte wichtig sind, wie z.B hohe Festigkeit bei gleichzeitiger Transluzenz, ist mit diesen Glaskeramiken jedoch nicht erzielbar.

Leucitfrei Glaskeramiken sind überdies aus der DD-PS 291 982 bekannt. Sie können Apatit enthalten und sind als Bestandteil von Glasionomerzementen für die Zahnmedizin verwendbar. Nachteilig ist aber ihr geringer thermischer Ausdehnungskoeffizient und ihre unzureichende Festigkeit, weshalb sie sich nicht als Dentalrestaurationsmaterial eignen. Das gilt auch für die in der DD-PS 242 216 beschriebenen Glaskeramiken, die als Kristallphasen Glimmer und Cordierit enthalten.

Neben den erwähnten leucitfreien Glaskeramiken sind auch leucithaltige Glaskeramiken bekannt, die allerdings keinen Phosphor enthalten. Derartige Materialien sind beispielsweise in der US-PS 4,604,366 und der US-PS 4,798,536 beschrieben. Auch wenn sich diese Glaskeramiken zu Dentalkronen verarbeiten lassen, so besitzen sich dennoch eine nur geringe Festigkeit und die aus ihnen hergestellten Dentalprodukte befriedigen hinsichtlich ihrer optischen und mechanisch-biologischen Eigenschaften, wie Biokompatibilität und Abrasionsverhalten, nicht.

Schließlich sind aus der EP-A-0 155 564 phosphatfreie leucithaltige Glaskeramiken auf Feldspatbasis bekannt. Durch Mischen verschiedener Glaskeramiken ist es möglich, einen gewünschten thermischen Ausdehnungkoeffizienten einzustellen.

Der Erfindung liegt die Aufgabe zugrunde, Glaskeramiken zu schaffen, die insbesondere infolge ihrer einfachen Verarbeitbarkeit, hohen Festigkeit, chemischen Beständigkeit und ihres vorteilhaften optischen Verhaltens als Dentalmaterialien und daraus geformte Dentalprodukte, wie vollkeramischen Dentalrestaurationen, z.B. Kronen, Brücken und künstlichen Zähnen, eingesetzt werden können.

Diese Aufgabe wird überraschenderweise durch die leucithaltige Phosphosilikat-Glaskeramik gemäß den Ansprüchen 1 bis 8 gelöst.

Gegenstand der vorliegenden Erfindung sind ebenfalls ein Verfahren zur Herstellung der Glaskeramik, die Verwendung der Glaskeramik sowie geformte Dentalprodukte die aus der Glaskeramik gebildet sind und demnach einen Gehalt an der Glaskeramik aufweisen.

Die erfindungsgemäße leucithaltige Phosphosilikat-Glaskeramik ist dadurch gekennzeichnet, daß sie die folgenden Komponenten enthält:

| | |
|---|---|
| SiO₂ | 49,0 - 57,5 Gew.-% |
| Al₂O₃ | 11,4 - 21,0 Gew.-% |
| P₂O₅ | 0,5 - 5,5 Gew.-% |
| CaO | 2,5 - 11,5 Gew.-% |
| K₂O | 9,0 - 22,5 Gew.-% |
| Na₂O | 1,0 - 9,5 Gew.-% |
| Li₂O | 0 - 2,5 Gew.-% |
| B₂O₃ | 0 - 2,0 Gew.-% |
| TiO₂ | 0 - 3,0 Gew.-% |
| ZrO₂ | 0,8 - 8,5 Gew.-% |
| CeO₂ | 0 - 3,0 Gew.-% |
| F | 0,25 - 2,5 Gew.-% |
| La₂O₃ | 0 - 3,0 Gew.-% |
| ZnO | 0 - 3,0 Gew.-% |
| BaO | 0 - 3,0 Gew.-% |
| MgO | 0 - 3,0 Gew.-% |
| SrO | 0 - 3,0 Gew.-% |

und daß sie eine Leucit-Kristallphase und mindestens eine weitere Kristallphase sowie eine oder mehrere Glasphasen enthält. Vorzugsweise besteht die Glaskeramik im wesentlichen aus den zuvor genannten Komponenten.

Es wurde völlig überraschend gefunden, daß es bei der gesteuerten Kristallisation von Ausgangsgläsern mit der vorstehend für die erfindungsgemäße Glaskeramik angegebenen chemischen Zusammensetzung neben der Bildung einer Leucit-Kristallphase auch zur Bildung mindestens einer weiteren Kristallphase, vorzugsweise einer phosphathaltige Kristallphase, kommt. Es wird angenommen, daß die vorteilhaften Eigenschaften der erfindungsgemäßen Glaskeramik insbesondere auf das gleichzeitige Vorliegen von Leucitkristallen, die vorzugsweise die Hauptkristallphase bilden, und weiteren Kristallen zurückzuführen sind.

Für einige der Komponenten der Glaskeramik existieren bevorzugte Mengenbereiche. Diese können unabhängig voneinander gewählt werden und sind wie folgt:

| | |
|---|---|
| SiO₂ | 50 - 57 Gew.-% |
| P₂O₅ | 0,5 - 4,0 Gew.-% |
| CaO | 2,5 - 7,0 Gew.-% |
| K₂O | 9,0 - 15,0 Gew.-% |
| Na₂O | 5,0 - 9,5 Gew.-% |
| Li₂O | 0 - 1,5 Gew.-% |
| B₂O₃ | 0 - 1,0 Gew.-% |
| TiO₂ | 0 - 2,5 Gew.-% |
| ZrO₂ | 0,8 - 5,0 Gew.-% |
| La₂O₃ | 0 - 2,0 Gew.-% |

Für die Eigenschaften der Glaskeramik und insbesondere die mechanische Festgkeit ist es vorteilhaft, wenn die Kristalle der einzelnen Phasen jeweils im wesentlichen gleich groß sind und die Kristalle aller Kristallphasen eine mittlere Größe von weniger als 5 µm, bevorzugt weniger als 3 µm, bezogen auf die Anzahl der Kristalle, aufweisen.

Es hat sich herausgestellt, daß besonders vorteilhafte Glaskeramiken solche sind, bei denen als Kristallphasen eine Leucit-Kristallphase und eine Kristallphase aus länglichen phosphathaltigen Kristallen vorhanden sind. Ganz besonders bevorzugt ist es, wenn die länglichen phosphathaltigen Kristalle nadelförmige Apatitkristalle sind, welche insbesondere eine mittlere Größe von weniger als 2 µm, bezogen auf die Anzahl der Kristalle, haben.

Zur Herstellung der erfindungsgemäßen Glaskeramik wird so vorgegangen, daß man
(a) ein Glas herstellt, welches die zuvor genannten Komponenten enthält,
(b) das Glas in Form eines Pulvers oder Granulats oder eines aus Pulver gepreßten Grünlings einer Wärmebehandlung im Temperaturbereich von 850 bis 1200°C für eine Dauer von 30 Minuten bis 4 Stunden, insbesondere 1 bis 2,5 Stunden, unter Bildung der Glaskeramik unterzieht und
(c) gegebenenfalls der gebildeten Glaskeramik einen Zusatzstoff zusetzt.

Insbesondere wird in Schritt (a) so vorgegangen, daß geeignete Ausgangsmaterialien, wie beispielsweise Oxide, Carbonate, Phosphate und Fluoride, in den gewünschten Gewichtsverhältnissen gemischt und 0,5 bis 4 Stunden lang bei einer Temperatur von 1400 bis 1700° C, insbesondere 1500 bis 1650° C, zu einer homogenen Glasschmelze erschmolzen werden. Das erschmolzene Glas wird danach in Wasser abgeschreckt (gefrittet), wodurch ein Granulat erhalten wird.

Der sich anschließende Verfahrensschritt (b) wird bevorzugt so ausgeführt, daß das erhaltenen Granulat getrocknet und auf eine gewünschte Partikelgröße aufgemahlen wird. Durch Veränderung der Partikelgröße ist es möglich, die Eigenschaften des schließlich gebildeten Glaskeramik zu verändern. So ist es beispielsweise möglich, durch Einsatz eines groben Granulats die Bildung von Leucit-Kristallen zu Gunsten der phosphathaltigen Kristalle zurückzudrängen. Eine vorteilhafte mittlere Partikelgröße liegt bei weniger als 90 µm und insbesondere weniger als 45 µm. Das gemahlene Glas wird dann der bereits erwähnten Wärmebehandlung unterworfen, wodurch die gewünschten Kristallphasen gebildet werden.

Es ist möglich, der Glaskeramik noch Zusatzstoffe, wie Farbstoffe und Fluoreszenzstoffe zuzusetzen. Geeignete Farbstoffe sind Farbpigmente, Oxide der 3d-Elemente oder Metallkolloide. Einsetzbare Fluoreszenzstoffe sind mit d- und/oder f-Elementen dotierte Yttriumsilikate.

Zur Veränderung von insbesondere den thermischen und optischen Eigenschaften der Glaskeramiken können diesen als Zusatzstoffe auch weitere Gläser, Keramiken, weitere Glaskeramiken, Trübungsstoffe und/oder Stabilisatoren zugegeben werden.

Die mikroheterogene Struktur der erfindungsgemäßen Glaskeramik, d.h. das Vorliegen von mindestens zwei unterschiedlichen Kristallphasen und mindestens einer Glasphase, wurde mittels Rasterelektronenmikroskopie festgestellt, und die gebildeten Kristalle wurden mittels Röntgendiffraktometrie identifiziert.

Bei einer Glaskeramik mit zwei Glasphasen kann infolge einer flüssig-flüssig-Phasentrennung die eine Glasphase in Form von Tropfen vorliegen, die in der durch die andere Glasphase gebildeten Matrix eingebettet sind. Eine solche Entmischung ist elektronenmikroskopisch sehr gut nachweisbar.

Die primäre oder Haupt-Kristallphase wird bevorzugt durch Leucitkristalle gebildet. Diese entstehen vermutlich während der erforderlichen Wärmebehandlung durch den Mechanismus der gesteuerten Oberflächenkristallisation an der Oberfläche der einzelnen Partikel des Ausgangsglases. Im Frühstadium der Kristallisation liegen die Leucitkristalle fast ausschließlich an den Korngrenzen der einzelnen Glaskörner vor, mit Fortschreiten der Kristallisation werden aber auch innerhalb der Glaskörner Leucitkristalle gebildet, so daß diese schließlich homogen über das gesamte Probenvolumen verteilt vorliegen. Die Größenverteilung der gebildeten Kristalle ist sehr eng.

Zusätzlich zu der Leucitausscheidung wird mindestens eine weitere, vorzugsweise eine phosphathaltige, insbesondere calciumphosphathaltige, und besonders bevorzugt eine apatithaltige Kristallphase gebildet. Der Apatit kann dabei z.B. als Hydroxyl- und/oder Fluor-Apatit vorliegen.

Mit Hilfe der röntgendiffraktometrischen Untersuchungen konnte gezeigt werden, daß die phosphathaltige Kristallphase zunächst in sphärischer Form ausgeschieden wird und sich mit zunehmender Temperatur und Temperzeit stäbchen- bzw. nadelförmige Kristalle bilden. Die genaue Form der ausgeschiedenen Kristalle hängt dabei unter anderem von dem CaO-, P₂O₅- und Fluor-Gehalt des Ausgangsglases ab. Es hat sich herausgestellt, daß Glaskeramiken, in denen zusätzlich zum Leucit phosphathaltige stäbchen- bzw. nadelförmiger Kristalle vorliegen, eine besonders hohe mechanische Festigkeit aufweisen.

Die angegebenen Mengenbereiche der Komponenten der erfindungsgemäßen Glaskeramik sind dabei erforderlich, um die Bildung von stäbchen- bzw. nadeligen phosphathaltigen Kristallen zu bewirken. Es hat sich gezeigt, daß auch bei niedrigen CaO- (2,5 Gew.-%) und P₂O₅-Gehalten (0,5 Gew.-%) neben dem Leucit eine aus länglichen Kristallen bestehende Kristallphase im Gefüge der Glaskeramik auftritt. Diese Kristalle sind jedoch aufgrund ihrer geringen Größe zum Teil röntgenamorph, so daß ihre Identität mittels Röntgendiffraktometrie nicht eindeutig bestimmt werden konnte. Aufgrund der identischen Form liegt jedoch die Vermutung nahe, daß es sich bei diesen Ausscheidungen ebenfalls um phosphathaltige Kristallphasen handelt.

Die in den erfindungsgemäßen Glaskeramiken enthaltenen Kristalle haben vorzugsweise eine mittlere Länge von maximal 3 µm, insbesondere weniger als 2 µm, und einen Durchmesser von weniger als 100 nm.

Zusätzlich zu den obengenannten Phasen können die erfindungsgemäßen Glaskeramiken noch weitere Kristallphasen, wie beispielsweise SiO₂- oder ZrO₂-Kristalle, aufweisen.

Die erfindungsgemäße Glaskeramik kann aufgrund ihrer hervorragenden Eigenschaften, insbesondere ihrer hohen Festigkeit und chemischen Beständigkeit, in vorteilhafter Weise als Dentalmaterial, wie z.B. Glaskeramikzement, oder Bestandteil davon eingesetzt werden.

Weiter können aus der Glaskeramik in einfacher Weise auch geformte Dentalprodukte gebildet werden oder sie als Bestandteile von solchen Dentalprodukten eingesetzt werden. Bevorzugte geformte Dentalprodukte sind vollkeramische oder metallkeramische Dentalrestaurationen, wie Kronen, Brücken, Teilkronen, Inlays, Onlays, künstliche Zähne, Stumpfaufbauten oder Facetten.

Besondere Vorteile zeigt die erfindungsgemäße Glaskeramik bei der Verarbeitung zu Dentalrestaurationen, die jeweils individuell für den einzelnen Patienten angepaßt werden müssen, wie z.B Brücken oder Kronen. Dabei ist es nämlich möglich, einen aus der Glaskeramik gebildeten Rohling im heißen viskosen Zustand selbst bei Temperaturen von unterhalb 1200° C zu der gewünschten Form zu verpressen. Bei dieser Verarbeitung kommt es im Gegensatz zu herkömmlichen Glaskeramiken auch nicht zu einer unerwünschten Reaktion mit der Einbettmasse, was einen bedeutenden Vorteil für den Dentaltechniker darstellt.

Zur Herstellung von geformten Dentalprodukten kann insbesondere wie folgt vorgegangen werden. Zunächst wird aus dem Ausgangsglas oder bereits hergestellter erfindungsgemäßer Glaskeramik ein Pulver mit einer Teilchengröße von vorzugsweise weniger als 90 µm gebildet. Dieses Pulver wird uniaxial bei Raumtemperatur trockengepreßt und dann bei einer Temperatur von 850 bis 1200°C, vorzugsweise 900 bis 1000° C, für 15 Minuten bis 2 Stunden, vorzugsweise 30 Minuten bis zu 1 Stunde, zusammengesintert. Der so erhaltene Glaskeramik-Rohling wird anschließend in einem Spezialpreßofen, wie er beispielsweise aus EP-A-0 231 773 bekannt ist, bei Temperaturen bis zu 1200°C, vorzugsweise von 950 bis 1150°C, in eine Hohlform gepreßt. Dieses Verfahren wird auch als "viskoses Fließen" bezeichnet. Die Form stellt dabei die gewünschte Dentalsuprastruktur, wie z.B. Krone, Teilkrone oder Brücke, dar. Nach Abkühlen und Entformen wird das gewünschte fertige Dentalprodukt erhalten.

Die erfindungsgemäßen Dentalprodukte können nach ihrer Herstellung auch noch einer thermischen Nachbehandlung unterworfen werden und/oder mit einer zusätzlichen Glasur-, Sinterkeramik- oder Glaskeramikschicht versehen werden. Diese zusätzlichen Behandlungen werden üblicherweise bei einer Temperatur von 700 bis 1000°C durchgeführt. Eine besonders hohe Festigkeit wird erzielt, wenn die Glaskeramik zunächst einer thermischen Zusatzbehandlung zur Ausbildung einer Eigenglasur unterworfen und anschließend mit einer weiteren Glasur- oder Glaskeramikschicht versehen wird. Die so erhaltenen erfindungsgemäßen Dentalprodukte weisen transluzente Eigenschaften und Biegefestigkeiten von bis zu 400 MPa auf.

Weiter können geformte Dentalprodukte auch durch Fräsen aus dem monolithischen Glaskeramik-Rohling hergestellt werden.

Schließlich können die erfindungsgemäßen Glaskeramiken auch in Pulverform mit z.B. Wasser vermischt und auf ein metallisches oder keramisches Substrat aufgebracht werden, wobei nach Formung und Brennen bei Temperaturen von 700 bis 1100°C eine fertige Dentalrestauration, wie z.B. eine Brücke, eine Krone, eine Teilkrone, eine Facette, ein Stumpfaufbau oder ein künstlicher Zahn für abnehmbare Prothesen, entsteht.

Zusammenfassend eignen sich die erfindungsgemäßen Glaskeramiken aufgrund ihrer vorteilhaften mechanischen, optischen, chemischen und biologischen Eigenschaften und ihrer Verarbeitungseigenschaften besonders für die Dentaltechnik, z.B. zur Herstellung von Dentalrestaurationen.

Im folgenden wird die Erfindung anhand von Beispielen näher erläutert.

### Beispiele

### Beispiele 1 bis 29

Es wurden insgesamt 29 verschiedene erfindungsgemäße Glaskeramiken hergestellt. Sie hatten die in der Tabelle I angegebenen chemischen Zusammensetzungen.

Für einige dieser Glaskeramiken sind in Tabelle II die jeweils eingesetzte Wärmebehandlungsmethode und etwaige zusätzliche Behandlungen sowie ausgewählte Eigenschaften angegeben.

Die Beispiele verdeutlichen wie durch Veränderung der chemischen Zusammensetzung des Ausgangsglases und des Herstellungsverfahrens Glaskeramiken mit unterschiedlichem Gefüge und Eigenschaften erhalten werden können.

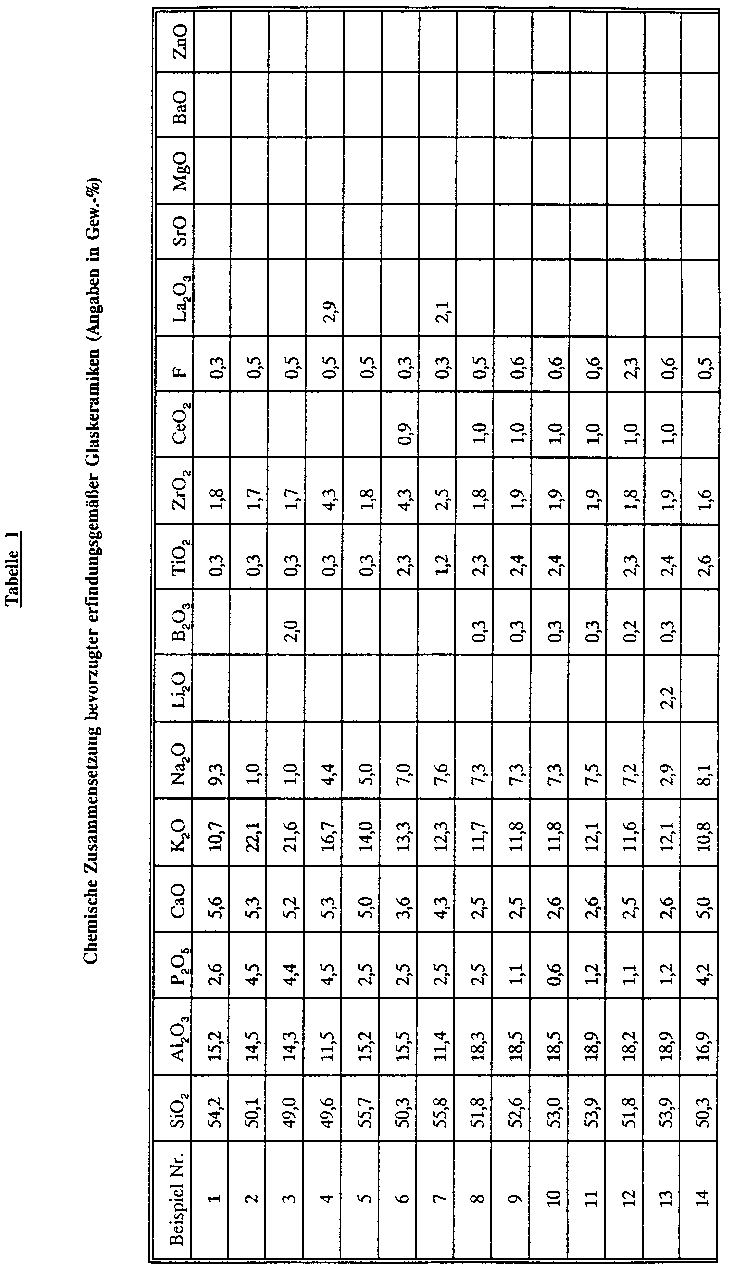

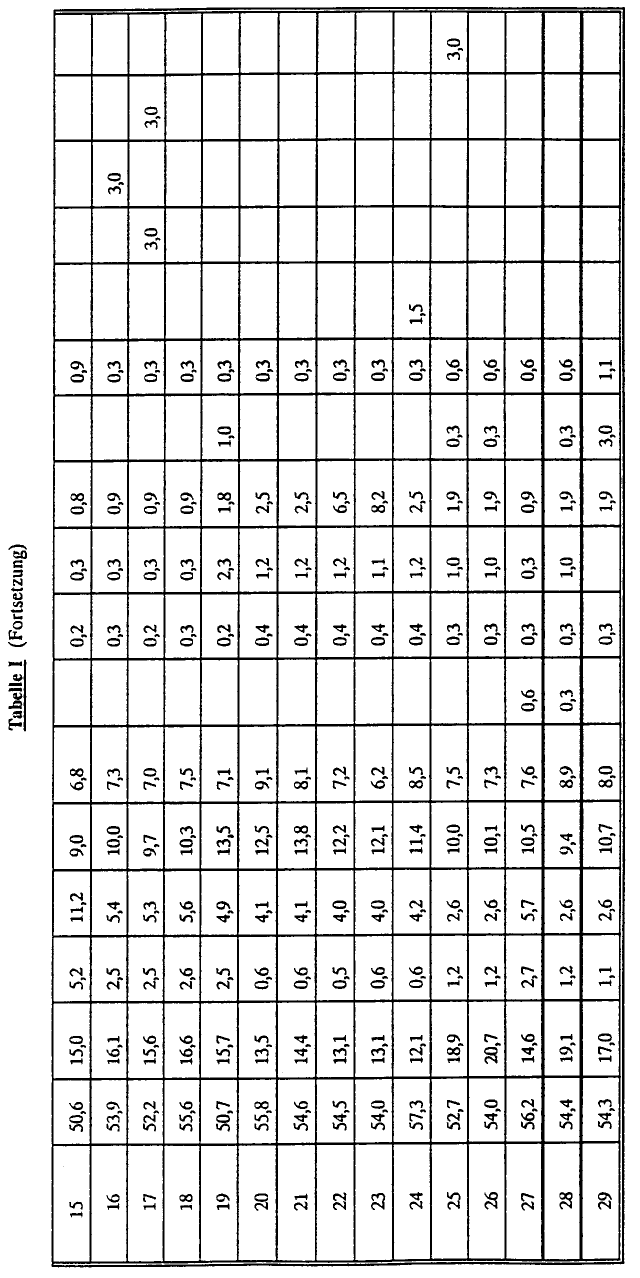

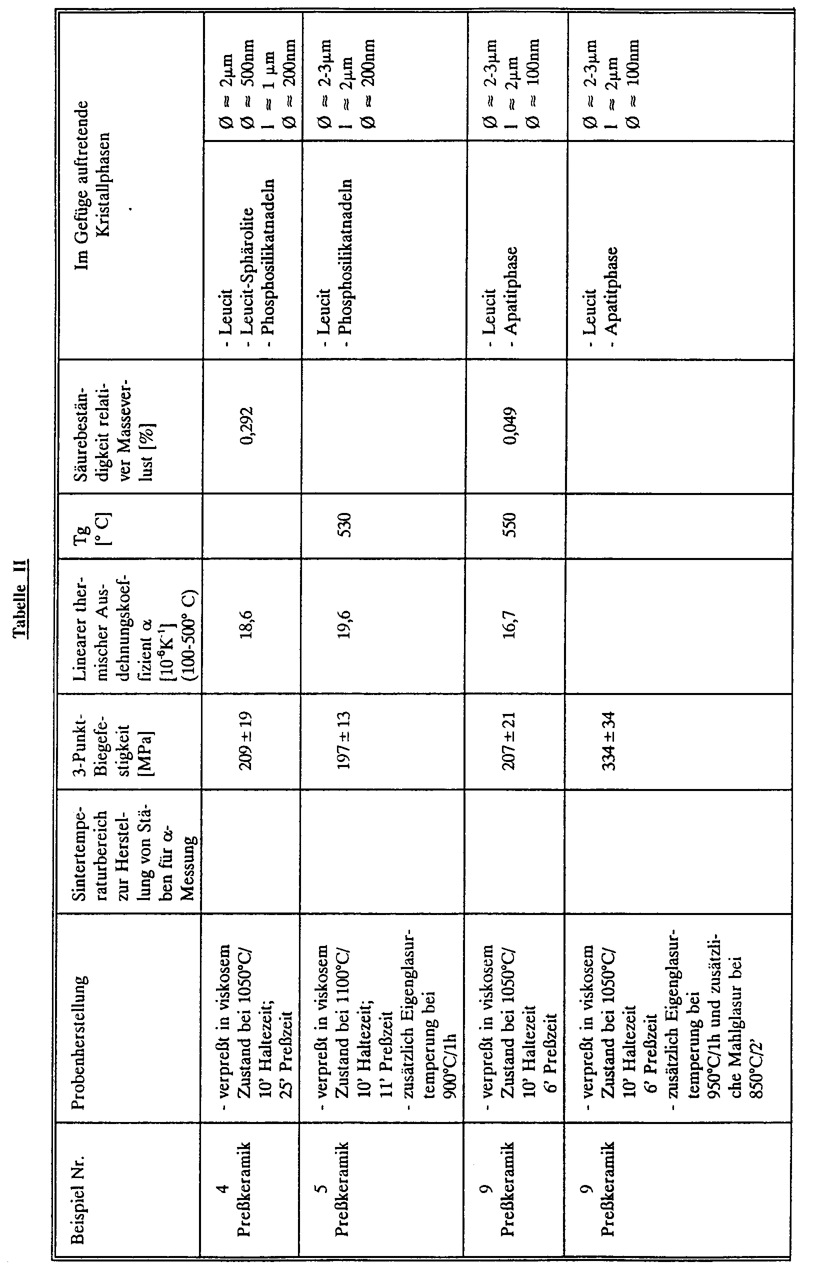

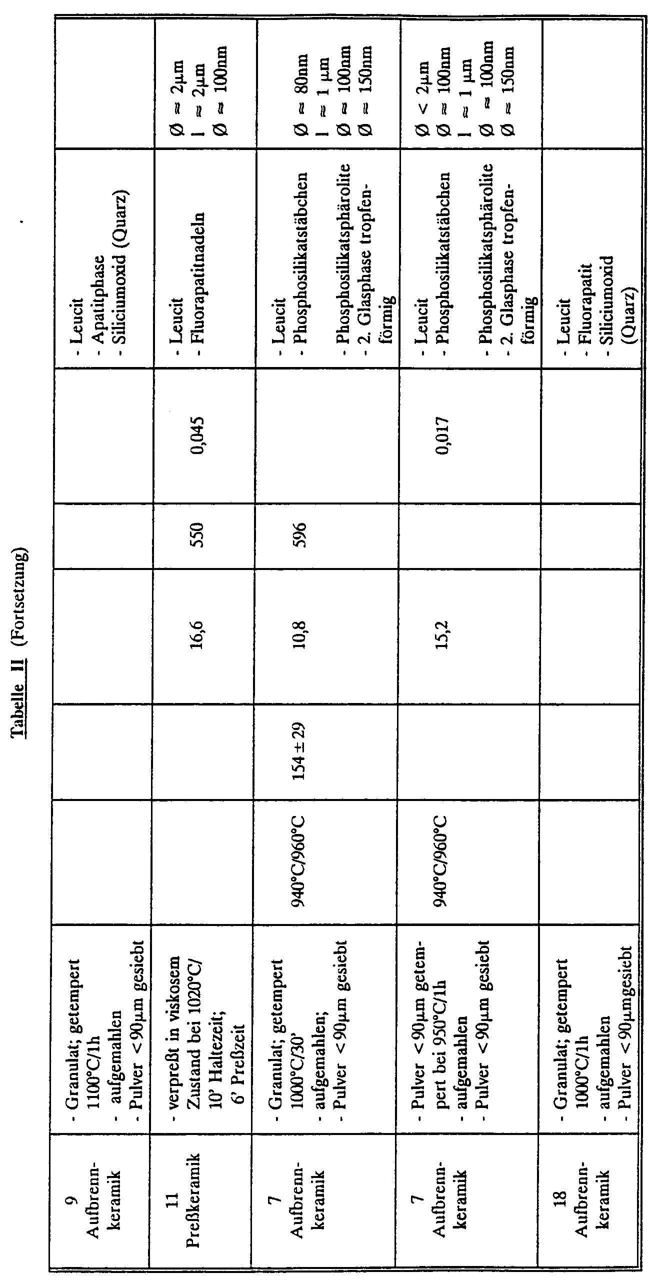

### Beispiel 30

Dieses Beispiel beschreibt die Herstellung einer erfindungsgemäßen Glaskeramik und deren Einsatzmöglichkeit als Gerüstmaterial zur Herstellung eines individuell formbaren vollkeramischen Produktes, wie z.B. eine Krone oder Brücke, auf welches zusätzlich eine angepaßte Dentalsinterkeramik aufgebrannt wird.

Zunächst wurde ein Ausgangsglas mit der in Tabelle I für Beispiel 4 angegebenen chemischen Zusammensetzung hergestellt. Dazu wurde ein Gemenge von Oxiden, Carbonaten, Phosphaten und Fluoriden in einem Platin/Rhodium-Tiegel bei einer Temperatur von 1500 bis 1640°C während einer Homogenisierungszeit von einer Stunde erschmolzen. Die Glasschmelze wurde in Wasser abgeschreckt, und die gebildete Glasfritte wurde getrocknet, in einer Achat-Kugelmühle 2 Stunden lang gemahlen und auf eine Korngröße von weniger als 45 µm gesiebt. Anschließend wurde das erhaltene Glaspulver mittels einer uniaxialen Trockenpresse bei Raumtemperatur und bei einem Preßdruck von 1000 bar zu zylindrischen Grünlingen mit einer Masse von je etwa 4 g gepreßt.

Die Grünlinge wurden dann im Vakuum in einem Brennofen zur erfindungsgemäßen Glaskeramik in Form eines Rohlings gesintert, wobei mit einer Aufheizrate von 30° C/Minute gearbeitet und die Endtemperatur von 950°C für 30 Minuten gehalten wurde.

Die erhaltenen Rohlinge wurden schließlich unter Verwendung des Preßverfahrens und Preßofens gemäß EP-A-0 231 773 unter Vakuum im viskosen Zustand in die für den jeweiligen Test gewünschte Probengeometrie verpreßt. Dabei betrugen die Bereitschaftstemperatur des Preßofens 600°C, die Heizrate bis zur Preßtemperatur 60°C/min, die Preßtemperatur 1100°C, die Haltezeit bei der Preßtemperatur 10 min und der Preßdruck 8 bar. Nach dem Preßvorgang wurde die Preßform an der Luft abgekühlt, und die Probenkörper wurden durch Sandstrahlen mit Glasperlen entformt.

Die erhaltenen Proben wurden folgenden Tests unterzogen:

### 3-Punkt-Biegefestigkeit

Hierzu wurden Stäbe mit den Maßen 1,5 x 4,8 x 20 mm gepreßt, und diese wurden mit SiC-Naßschleifpapier (1000-er Körnung) allseitig überschliffen. Die Ermittlung der Biegefestigkeit erfolgte bei einer Stützweite des Prüfmittels von 15 mm und einer Vorschubgeschwindigkeit der Lastaufbringung von 0,5 mm/min. Die bei diesen Bedingungen gemäß ISO 6872-1984 ermittelte 3-Punkt-Biegefestigkeit betrug 209 ± 19 MPa.

Auf einen Teil der gepreßten Stäbe wurde nach dem Schleifvorgang eine Dentalkeramik mit angepaßtem thermischen Ausdehnungskoeffizienten in einer bei der Herstellung von Teilkronen, Kronen oder Brücken üblichen Weise aufgebrannt. Nach 5-maliger Wiederholung des Aufbrennvorgangs hatte die Aufbrennglasur jeweils eine Schichtstärke von ca. 20 bis 30 µm. Für die so behandelten Stäbe wurde unter den oben angegebenen Bedingungen eine Biegefestigkeit von 224 ± 10 MPa bestimmt.

### Linearer thermischer Ausdehnungskoeffizient

Hierzu wurden zylindrische Proben mit einem Durchmesser von 6 mm und einer Länge von 26 mm gepreßt. Der im Temperaturbereich von 100 bis 500°C für diese Proben bestimmte Ausdehnungskoeffizient betrug 18,6 x 10⁻⁶K⁻¹.

### Säurebeständigkeit

Hierzu wurden scheibenförmige Proben mit einem Durchmesser von 15 mm und einer Dicke von 1,5 mm gepreßt und anschließend mit SiC-Naßschleifpapier (1000er Körnung) allseitig überschliffen. Der gemäß ISO 6872-1984 bestimmte relative Masseverlust dieser Proben nach 16-stündiger Lagerung in 4 Vol.-%iger wäßriger Essigsäurelösung betrug lediglich 0,292% und liegt damit deutlich unter dem Normwert für Dentalkeramikmaterialien von 0,5 %.

### Beispiel 31

Ebenso wie Beispiel 30 zeigt dieses Beispiel die Herstellung einer erfindungsgemäßen Glaskeramik und deren Einsatzmöglichkeit als Gerüstmaterial zur Herstellung eines individuell formbaren vollkeramischen Produktes, wie z.B. eine Krone oder Brücke.

Es wurden gepreßte Glaskeramik-Proben mit der in Tabelle I für Beispiel 9 angegebenen chemischen Zusammensetzung hergestellt. Dazu wurde die in Beispiel 30 beschriebene Verfahrensweise mit folgenden Änderungen gewählt:
- Sinterungsprozeß der Grünlinge zu den Glaskeramik-Rohlingen bei einer Endtemperatur von 920°C;
- Heizrate bis zur Preßtemperatur: 60°C/min;
- Preßtemperatur: 1050°C;
- Preßdruck: 5 bar.

Die entsprechend Beispiel 30 durchgeführten Test ergaben folgende Ergebnisse:

### 3-Punkt-Biegefestigkeit

Sie wurde für unterschiedliche Proben bestimmt:
- Proben (a):: verpreßt ohne weitere Behandlung: 207 ± 21 MPa
- Proben (b):: Sie wurden nach dem Schleifvorgang einer 1-stündigen Temperung bei 950°C an der Luft unterzogen und wiesen daraufhin eine Eigenglasur auf der Oberfläche auf. Sie hatten eine merklich höhere Biegefestigkeit von 260 ± 21 MPa.
- Proben (c):: Auf sie wurde nach dem Schleifvorgang bei 850°C und einer Haltezeit von 2 min eine Dentalkeramik mit angepaßtem Ausdehnungskoeffizienten in üblicher Weise aufgebrannt. Nach 5-maliger Wiederholung des Aufbrennvorgangs hatte die Aufbrennglasur eine Schichtstärke von ca. 20 bis 30 µm. Für die so behandelten Proben wurde eine Biegefestigkeit von 260 ± 65 MPa bestimmt.
- Proben (d):: Sie wurden durch Temperung wie bei den Proben (b) und anschließendes Aufbrennen einer Dentalkeramik wie bei den Proben (c) erhalten. Die Summe der Schichtstärke von Eigenglasur und Aufbrennglasur betrug ca. 50 µm. Für die so behandelten Proben wurde eine ausgezeichnete Biegefestigkeit von 334 ± 34 MPa bestimmt.

### Linearer thermischer Ausdehnungskoeffizient

Hierzu wurden Proben eingesetzt, die thermisch nicht weiter behandelt worden waren, und sie hatten einen Ausdehnungskoeffizienten von 16,7 x 10⁻⁶K⁻¹.

### Säurebeständigkeit

Hierzu wurden Proben eingesetzt, die nach dem Preßvorgang thermisch nicht weiter behandelt worden waren, und sie zeigten einen relativen Masseverlust von 0,049 %.

### Beispiel 32

Dieses Beispiel beschreibt die Herstellung einer erfindungsgemäßen Glaskeramik, die aufgrund ihrer Transluzenz als Teilkrone oder als Gerüstmaterial für vollkeramische Dentalprodukte, wie Kronen oder Brücken, eingesetzt werden kann.

Hierzu wurden gepreßte Glaskeramik-Proben mit der in Tabelle I für Beispiel 11 angegebenen chemischen Zusammensetzung hergestellt. Dazu wurde die in Beispiel 30 beschriebene Verfahrensweise mit folgenden Änderungen gewählt:
- Sinterungsprozeß der Grünlinge zu den Glaskeramik-Rohlingen bei einer Endtemperatur von 920°C;
- Preßtemperatur: 1020°C;
- Preßdruck: 5 bar.

Die entsprechend Beispiel 30 durchgeführten Tests führten zu folgenden Ergebnisse:

### 3-Punkt-Biegefestigkeit

Sie wurde für verschiedene Proben bestimmt:
- Proben (a):: ohne weitere thermische Behandlung: 154 ± 29 MPa
- Proben (b):: Sie wurden nach dem Schleifvorgang einer 1-stündigen Temperung bei 900°C an der Luft unterzogen und wiesen daraufhin eine Eigenglasur auf der Oberfläche auf. Sie hatten eine deutlich höhere Biegefestigkeit von 208 ± 66 MPa.
- Proben (c):: Auf sie wurden nach dem Schleifvorgang bei 850°C und einer Haltezeit von 2 min eine Dentalkeramik mit angepaßtem Ausdehnungskoeffizienten in üblicher Weise aufgebrannt. Nach 5-maliger Wiederholung des Aufbrennvorgangs hatte die Aufbrennglasur eine Schichtstärke von ca. 20 bis 30 µm. Für die so behandelten Proben wurde eine Biegefestigkeit von 290 ± 34 MPa bestimmt.

### Linearer thermischer Ausdehnungskoeffizient

Hierzu wurden Proben eingesetzt, die thermisch nicht weiter behandelt worden waren, und sie hatten einen Ausdehnungskoeffizienten von 16,6 x 10⁻⁶K⁻¹.

### Säurebeständigkeit

Hierzu wurden Proben eingesetzt, die nach dem Preßvorgang thermisch nicht weiter behandelt worden waren, und sie zeigten einen relativen Masseverlust von 0,045 %.

### Beispiel 33

Diese Beispiel beschreibt die Herstellung einer erfindungsgemäßen Glaskeramik, die sich zum Aufbrennen auf ein Metallgerüst eignet, und somit als Bestandteil von metallkeramischen Dentalprodukten, wie metallkeramischen Kronen oder Brücken, eingesetzt werden kann.

Zunächst wurde ein Ausgangsglas mit der in Tabelle I für Beispiel 7 angegebenen chemischen Zusammensetzung hergestellt. Dazu wurde ein Gemenge von Oxiden, Carbonaten, Phosphaten und Fluoriden in einem Platin/Rhodium-Tiegel bei einer Temperatur von 1500 bis 1650°C während einer Homogenisierungszeit von 1 bis 1,5 Stunden erschmolzen. Die Glasschmelze wurde in Wasser abgeschreckt, und das gebildete Glasgranulat wurde getrocknet. Ein Teil des gebildeten Granulats wurde zu einem Glaspulver mit einer mittleren Korngröße von weniger als 90 µm gemahlen.

Zur Bildung von Glaskeramiken aus dem Ausgangsglas wurde das Glasgranulat 0,5 Stunden lang bei 1000°C und das Glaspulver 1 Stunde lang bei 950°C wärmebehandelt. Die jeweils gebildete Glaskeramik wurde mittels Rasterelektronenmikroskopie untersucht und die gebildeten Kristalle wurden mittels Röntgendiffraktometrie identifiziert. In beiden Glaskeramiken konnten eine Leucit-Kristallphase und eine phosphathaltige Kristallphase nachgewiesen werden.

Die aus dem Glasgranulat und dem Glaspulver gebildeten Glaskeramiken wurden jeweils wieder aufgemahlen und zu stäbchenförmigen Grünkörpern in einem Vakuumofen bei einer Aufheizgeschwindigkeit von 60°C/min und einer Haltezeit von 1 min bei 960 bis 980°C gesintert. Für die so erhaltenen Proben sowie für einem aus dem Ausgangsglas unter gleichen Bedingungen hergestellten Grünkörper wurden folgende thermische Ausdehnungskoeffizienten im Temperaturbereich von 100 bis 500°C bestimmt:
- Glaskeramik hergestellt aus Glasgranulat : 10,8 x 10⁻⁶K⁻¹
- Glaskeramik hergestellt aus Glaspulver : 15,2 x 10⁻⁶K⁻¹
- Ausgangsglas : 10,7 x 10⁻⁶K⁻¹

Durch geeignetes Mischen dieser drei Materialien läßt sich der Ausdehnungskoeffizent so einstellen, daß die erhaltene Dentalkeramik zum Aufsintern auf eine Dentallegierung verwendet werden und damit zur Bildung einer metallkeramischen Dentalrestauration eingesetzt werden kann.

In analoger Weise lassen sich zur Erzielung von gewünschten Ausdehnungskoeffizienten auch unterschiedliche erfindungsgemäße Glaskeramiken miteinander oder mit Ausgangsgläser mischen. So ist es z.B. möglich, die Glaskeramik gemäß Beispiel 7 mit einem Ausgangsglas der chemischen Zusammensetzung gemäß Beispiel 24 in einem Verhältnis von 70 : 30 Gew.-% zu mischen, um somit eine erfindungsgemäße Glaskeramik mit hervorragenden optischen Eigenschaften und einem Ausdehnungskoeffizienten von z.B. 13,0 x 10⁻⁶K⁻¹ zu erhalten. Eine solche Glaskeramik eignet sich ausgezeichnet als Aufbrennkeramik für eine Dentallegierung.

### Beispiel 34

Diese Beispiel beschreibt eine erfindungsgemäße Glaskeramik, die in Pulverform durch Zugabe von organischen Plastifizierungsmitteln in einen plastischen Zustand überführbar ist und sich durch geeignete Formgebungsverfahren zu Dentalprodukten, wie künstlichen Zähnen, verarbeiten läßt.

Zunächst wurde ein Ausgangsglas mit der in Tabelle I für Beispiel 22 angegebenen chemischen Zusammensetzung hergestellt. Dazu wurde entsprechend dem Verfahren gemäß Beispiel 33 ein Glaspulver mit einer mittleren Korngröße von weniger als 90 µm hergestellt. Dann wurde das Glaspulver bei einer Temperatur von 1050° C 1 Stunde lang getempert. In der dadurch gebildeten Glaskeramik konnten Leucitkristalle und phosphathaltige Kristalle nachgewiesen werden.

Die aus dem Glaspulver gebildete Glaskeramik wurde wieder aufgemahlen und zu Prüfkörpern verarbeitet. Der Ausdehnungskoeffizient sowie die Säurebeständigkeit wurden dann entsprechend Beispiel 32 mit den folgenden Ergebnissen bestimmt:
- Ausdehnungskoeffizient: 14,1 x 10⁻⁶K⁻¹
- Relativer Masseverlust: 0,017%.

## Patentansprüche

1. Leucithaltige Phosphosilikat-Glaskeramik, **dadurch gekennzeichnet**, daß sie die folgenden Komponenten enthält:
| | |
|---|---|
| SiO₂ | 49,0 - 57,5 Gew.-% |
| Al₂O₃ | 11,4 - 21,0 Gew.-% |
| P₂O₅ | 0,5 - 5,5 Gew.-% |
| CaO | 2,5 - 11,5 Gew.-% |
| K₂O | 9,0 - 22,5 Gew.-% |
| Na₂O | 1,0 - 9,5 Gew.-% |
| Li₂O | 0 - 2,5 Gew.-% |
| B₂O₃ | 0 - 2,0 Gew.-% |
| TiO₂ | 0 - 3,0 Gew.-% |
| ZrO₂ | 0,8 - 8,5 Gew.-% |
| CeO₂ | 0 - 3,0 Gew.-% |
| F | 0,25 - 2,5 Gew.-% |
| La₂O₃ | 0 - 3,0 Gew.-% |
| ZnO | 0 - 3,0 Gew.-% |
| BaO | 0 - 3,0 Gew.-% |
| MgO | 0 - 3,0 Gew.-% |
| SrO | 0 - 3,0 Gew.-% |
und daß sie eine Leucit-Kristallphase und mindestens eine weitere Kristallphase sowie eine oder mehrere Glasphasen enthält.

2. Glaskeramik nach Anspruch 1, **dadurch gekennzeichnet**, daß die Mengen von einigen Komponenten unabhängig voneinander wie folgt sind:
| | |
|---|---|
| SiO₂ | 50 - 57 Gew.-% |
| P₂O₅ | 0,5 - 4,0 Gew.-% |
| CaO | 2,5 - 7,0 Gew.-% |
| K₂O | 9,0 - 15,0 Gew.-% |
| Na₂O | 5,0 - 9,5 Gew.-% |
| Li₂O | 0 - 1,5 Gew.-% |
| B₂O₃ | 0 - 1,0 Gew.-% |
| TiO₂ | 0 - 2,5 Gew.-% |
| ZrO₂ | 0,8 - 5,0 Gew.-% |
| La₂O₃ | 0 - 2,0 Gew.-% |

3. Glaskeramik nach Anspruch 1 oder 2, **dadurch gekennzeichnet**, daß sie eine phosphathaltige Kristallphase als weitere Kristallphase aufweist.

4. Glaskeramik nach Anspruch 3, **dadurch gekennzeichnet**, daß die phosphathaltige Kristallphase eine Apatit-Kristallphase ist.

5. Glaskeramik nach einem der Ansprüche 1 bis 4, **dadurch gekennzeichnet**, daß die Kristalle der einzelnen Phasen jeweils im wesentlichen gleich groß sind und die Kristalle aller Kristallphasen eine mittlere Größe von weniger als 5 µm, bevorzugt weniger als 3µm, bezogen auf die Anzahl der Kristalle, aufweisen.

6. Glaskeramik nach einem der Ansprüche 1 bis 5, **dadurch gekennzeichnet**, daß als Kristallphasen eine Leucit-Kristallphase und eine Kristallphase aus länglichen phosphathaltigen Kristallen vorhanden sind.

7. Glaskeramik nach Anspruch 6, **dadurch gekennzeichnet**, daß die länglichen phosphathaltigen Kristalle nadelförmige Apatitkristalle sind, welche bevorzugt eine mittlere Größe von weniger als 2 µm, bezogen auf die Anzahl der Kristalle, haben.

8. Glaskeramik nach einem der Ansprüche 1 bis 7, **dadurch gekennzeichnet**, daß sie als Zusatzstoffe Farbstoffe, Fluoreszenzstoffe, weitere Gläser, Keramiken, weitere Glaskeramiken, Trübungsstoffe und/oder Stabilisatoren enthält.

9. Verfahren zur Herstellung der Glaskeramik gemäß einem der Ansprüche 1 bis 8, **dadurch gekennzeichnet**, daß man
(a) ein Glas herstellt, welches die Komponenten gemäß Anspruch 1 enthält,
(b) das Glas in Form eines Pulvers oder Granulats oder eines aus Pulver gepreßten Grünlings einer Wärmebehandlung im Temperaturbereich von 850 bis 1200°C für eine Dauer von 30 Minuten bis 4 Stunden unter Bildung der Glaskeramik unterzieht und
(c) gegebenenfalls der gebildeten Glaskeramik einen Zusatzstoff zusetzt.

10. Verfahren nach Anspruch 9, **dadurch gekennzeichnet**, daß man die Wärmebehandlung in Schritt (b) für eine Dauer von 1 bis 2,5 Stunden durchführt.

11. Verfahren nach Anspruch 9 oder 10, **dadurch gekennzeichnet**, daß man in Schritt (b) das gebildete Glas vor der Wärmebehandlung auf eine mittlere Teilchengröße von weniger als 90 µm, bezogen auf die Anzahl der Teilchen, pulverisiert oder granuliert.

12. Verwendung der Glaskeramik gemäß einem der Ansprüche 1 bis 8 als (a) Dentalmaterial oder daraus geformtes Dentalprodukt oder (b) als Bestandteil von Dentalmaterialien oder von daraus geformten Dentalprodukten.

13. Verwendung nach Anspruch 12, wobei das geformte Dentalprodukt ein Krone, eine Brücke, eine Teilkrone, ein Inlay, ein Onlay, ein künstlicher Zahn, ein Stumpfaufbau oder eine Facette ist.

14. Verwendung nach Anspruch 12 oder 13, wobei das geformte Dentalprodukt ein Vollkeramik- oder Metallkeramik-Dentalprodukt ist.

15. Geformtes Dentalprodukt, welches einen Gehalt an der Glaskeramik gemäß einem der Ansprüche 1 bis 8 aufweist.

## Claims

1. Leucite-containing phosphosilicate glass ceramic, characterized in that it contains the following components:
| | |
|---|---|
| SiO₂ | 49.0 - 57.5% by weight |
| Al₂O₃ | 11.4 - 21.0% by weight |
| P₂O₅ | 0.5 - 5.5% by weight |
| CaO | 2.5 - 11.5% by weight |
| K₂O | 9.0 - 22.5% by weight |
| Na₂O | 1.0 - 9.5% by weight |
| Li₂O | 0 - 2.5% by weight |
| B₂O₃ | 0 - 2.0% by weight |
| TiO₂ | 0 - 3.0% by weight |
| ZrO₂ | 0.8 - 8.5% by weight |
| CeO₂ | 0 - 3.0% by weight |
| F | 0.25 - 2.5% by weight |
| La₂O₃ | 0 - 3.0% by weight |
| ZnO | 0 - 3.0% by weight |
| BaO | 0 - 3.0% by weight |
| MgO | 0 - 3.0% by weight |
| SrO | 0 - 3.0% by weight |
and that it contains a leucite crystalline phase and at least one further crystalline phase plus one or more glass phases.

2. Glass ceramic according to Claim 1, characterized in that the amounts of some components are, independently of one another, as follows:
| | |
|---|---|
| SiO₂ | 50 - 57.0% by weight |
| P₂O₅ | 0.5 - 4.0% by weight |
| CaO | 2.5 - 7.0% by weight |
| K₂O | 9.0 - 15.0% by weight |
| Na₂O | 5.0 - 9.5% by weight |
| Li₂O | 0 - 1.5% by weight |
| B₂O₃ | 0 - 1.0% by weight |
| TiO₂ | 0 - 2.5% by weight |
| ZrO₂ | 0.8 - 5.0% by weight |
| La₂O₃ | 0 - 2.0% by weight |

3. Glass ceramic according to Claim 1 or 2, characterized in that it has a phosphate-containing crystalline phase as further crystalline phase.

4. Glass ceramic according to Claim 3, characterized in that the phosphate-containing crystalline phase is an apatite crystalline phase.

5. Glass ceramic according to any of Claims 1 to 4, characterized in that the crystals of the individual phases are in each case essentially equal in size and the crystals of all crystalline phases have a mean size of less than 5 µm, preferably less than 3 µm, based on the number of crystals.

6. Glass ceramic according to any of Claims 1 to 5, characterized in that a leucite crystalline phase and a crystalline phase comprising elongated phosphate-containing crystals are present as crystalline phases.

7. Glass ceramic according to Claim 6, characterized in that the elongated phosphate-containing crystals are acicular apatite crystals which preferably have a mean size of less than 2 µm, based on the number of crystals.

8. Glass ceramic according to any of Claims 1 to 7, characterized in that it contains as additives dyes, fluorescent materials, further glasses, ceramics, further glass ceramics, opacifiers and/or stabilizers.

9. Process for producing the glass ceramic according to any of Claims 1 to 8, characterized in that
(a) a glass containing the components according to Claim 1 is produced,
(b) the glass in the form of a powder or granules or a green body pressed from powder is subjected to heat treatment in the temperature range from 850 to 1200°C for a period of from 30 minutes to 4 hours to form the glass ceramic and
(c) if desired, an additive is added to the glass ceramic formed.

10. Process according to Claim 9, characterized in that the heat treatment in step (b) is carried out for a period of from 1 to 2.5 hours.

11. Process according to Claim 9 or 10, characterized in that, in step (b), the glass formed is pulverized or granulated to a mean particle size of less than 90 µm, based on the number of particles, prior to heat treatment.

12. Use of the glass ceramic according to any of Claims 1 to 8 as (a) dental material or dental product moulded therefrom or (b) as a constituent of dental materials or of dental products moulded therefrom.

13. Use according to Claim 12, wherein the moulded dental product is a crown, a bridge, a part-crown, an inlay, an onlay, an artificial tooth, a stump build-up or a facing.

14. Use according to Claim 12 or 13, wherein the moulded dental product is an all-ceramic or metal-ceramic dental product.

15. Moulded dental product having a content of the glass ceramic according to any of Claims 1 to 8.

## Revendications

1. Vitocéramique de phosphosilicate contenant de la leucite, caracterisée en ce qu'elle contient les composants suivants :
| | |
|---|---|
| SiO₂ | 49,0 - 57,5 % en poids |
| Al₂O₃ | 11,4 - 21,0 % en poids |
| P₂O₅ | 0,5 - 5,5 % en poids |
| CaO | 2,5 - 11,5 % en poids |
| K₂O | 9,0 - 22,5 % en poids |
| Na₂O | 1,0 - 9,5 % en poids |
| Li₂O | 0 - 2,5 % en poids |
| B₂O₃ | 0 - 2,0 % en poids |
| TiO₂ | 0 - 3,0 % en poids |
| ZrO₂ | 0,8 - 8,5 % en poids |
| CeO₂ | 0 - 3,0 % en poids |
| F | 0,25 - 2,5 % en poids |
| La₂O₃ | 0 - 3,0 % en poids |
| ZnO | 0 - 3,0 % en poids |
| BaO | 0 - 3,0 % en poids |
| MgO | 0 - 3,0 % en poids |
| SrO | 0 - 3,0 % en poids |
et en ce qu'elle comprend une phase cristalline de leucite et au moins une autre phase cristalline ainsi qu'une ou plusieurs phases vitreuses.

2. Vitrocéramique selon la revendication 1, caractérisée en ce que les quantités de certains composants, indépendamment les uns des autres, sont les suivantes :
| | |
|---|---|
| SiO₂ | 50 - 57 % en poids |
| P₂O₅ | 0,5 - 4,0 % en poids |
| CaO | 2,5 - 7,0 % en poids |
| K₂O | 9,0 - 15,0 % en poids |
| Na₂O | 5,0 - 9,5 % en poids |
| Li₂O | 0 - 1,5 % en poids |
| B₂O₃ | 0 - 1,0 % en poids |
| TiO₂ | 0 - 2,5 % en poids |
| ZrO₂ | 0,8 - 5,0 % en poids |
| La₂O₃ | 0 - 2,0 % en poids |

3. Vitrocéramique selon la revendication 1 ou 2, caractérisée en ce qu'elle présente une phase cristalline contenant du phosphate comme phase cristalline supplémentaire.

4. Vitrocéramique selon la revendication 3, caractérisée en ce que la phase cristalline contenant du phosphate est une phase cristalline d'apatite.

5. Vitrocéramique selon une des revendications 1 à 4, caractérisée en ce que les cristaux de chaque phase sont essentiellement de même taille et que les cristaux de toutes les phases ont une taille moyenne inférieure à 5 µm, de préférence inférieure à 3 µm, par rapport au nombre des cristaux.

6. Vitrocéramique selon une des revendications 1 à 5, caractérisée en ce que, comme phases cristallines, une phase cristalline de leucite et une phase cristalline de cristaux allongés contenant du phosphate sont présentes.

7. Vitrocéramique selon la revendication 6, caractérisée en ce que les cristaux allongés contenant du phosphate sont des cristaux d'apatite en forme d'aiguille qui ont une taille moyenne de préférence inférieure à 2 µm, par rapport au nombre des cristaux.

8. Vitrocéramique selon une des revendication 1 à 7, caractérisée en ce qu'elle contient comme additifs des colorants, des agents fluorescents, d'autres vertes, des céramiques, d'autres vitrocéramiques, des agents opacifiants et/ou des stabilisants.

9. Procédé pour la préparation des vitrocéramiques selon une des revendications 1 à 8, caractérisé en ce que
a) on prépare un verre qui contient les composants selon la revendication 1,
b) on soumet le verte, sous forme d'une poudre ou d'un granulat ou d'un comprimé cru obtenu par compression de poudre, à un traitement thermique dans un domaine de température de 850 à 1200 °C, pendant 30 min à 4 heures, pour former la vitrocéramique et
c) on ajoute éventuellement un additif à la vitrocéramique formée.

10. Procédé selon la revendication 9, caractérisé en ce que le traitement thermique de l'étape b) dure de 1 à 2,5 heures.

11. Procédé selon la revendication 9 ou 10, caractérisé en ce que le verre formé à l'étape b) est mis sous forme de poudre ou de granulat de particules de taille moyenne inférieure à 90 µm, par rapport au nombre des particules, avant le traitement thermique.

12. Utilisation de la vitrocéramique selon une des revendications 1 à 8, comme (a) matériau dentaire ou produit dentaire façonné à partir de celle-ci ou (b) comme composant de matériaux dentaires ou de produits dentaires façonnés à partir de celle-ci.

13. Utilisation selon la revendication 12, pour laquelle le produit dentaire façonné est une couronne, un bridge, une couronne partielle, un inlay, un onlay, une dent artificielle, un appareil de recouvrement de moignons dentaires ou une facette.

14. Utilisation selon la revendication 12 ou 13, pour laquelle le produit dentaire façonné est un produit dentaire entièrement en céramique ou métallocéramique.

15. Produit dentaire façonné qui contient la vitrocéramique selon une des revendications 1 à 8.
